Europäisches Patentamt

European Patent Office

Office européen des brevets

(19)

(11) Publication number : **0 457 498 A2**

## (12) EUROPEAN PATENT APPLICATION

(21) Application number : 91304180.2

(22) Date of filing : 09.05.91

(51) Int. Cl.⁵ : **C12N 15/32, C12P 21/02, C12N 1/20, A01N 63/00**

The applicant has filed a statement in accordance with Rule 28 (4) EPC (issue of a sample only to an expert). Accession number(s) of the deposit(s): NRRL B-18644, NRRL B-18693 and NRRL B-18515.

(30) Priority : **15.05.90 US 524255**
**01.10.90 US 590903**

(43) Date of publication of application :
**21.11.91 Bulletin 91/47**

(84) Designated Contracting States :
**AT BE CH DE DK ES FR GB GR IT LI LU NL SE**

(71) Applicant : **Mycogen Corporation**
**5451 Oberlin Drive**
**San Diego, CA 92121 (US)**

(72) Inventor : **Sick, August J.**
**3188 San Helena Drive**
**Oceanside, California 92045 (US)**

(74) Representative : **Perry, Robert Edward et al**
**GILL JENNINGS & EVERY 53-64 Chancery Lane**
**London WC2A 1HN (GB)**

(54) Bacillus thuringiensis genes encoding dipteran-active toxins.

(57) Novel Bacillus thuringiensis genes encoding toxins which are active against dipteran insects have been cloned from a novel dipteran-active B. thuringiensis microbe. The DNA encoding the B. thuringiensis toxins can be used to transform various prokaryotic and eukaryotic microbes to express the B. thuringiensis toxins. These recombinant microbes, as well as the novel B. thuringiensis microbe itself, can be used to control dipteran insects in various environments.

EP 0 457 498 A2

Background of the Invention

Bacillus thuringiensis (B.t.) produces an insect tin designated as δ-endotoxin. It is synthesized by the B.t. sporulating cell. The toxin, upon being ingested in its crystalline form by susceptible insect larvae, is transformed into biologically active moieties by the insect gut juice proteases. The primary target is insect cells of the gut epithelium, which is rapidly destroyed.

The reported acivity spectrum of B.t. covers insect species within the orders Lepidoptera and Coleoptera, many of which are major pests in agriculture and forestry. The activity spectrum also includes the insect order Diptera, which includes mosquitoes and black flies. See Couch (1980) "Mosquito Pathogenicity of Bacillus thuringiensis var. israelensis," Developments in Industrial Microbiology 22:61-76; and Beegle (1978) "Use of Entomogenous Bacteria in Agroecosystems," Developments in Industrial Microbiology 20:97-104.

Brief Summary of the Invention

The subject invention concerns novel Bacillus thuringiensis (B.t.) genes which encode novel dipteran-active toxins. The novel genes of the invention are isolated from the novel B.t. isolate, known herein as Bacillus thuringiensis var. morrisoni PS71M3 (B.t. PS71M3). The toxins encoded by the novel genes of the invention have shown 100% mortality of Aedes aegypti and Culex pipiens after a 24-hour period.

Detailed Disclosure of the Invention

The genes of the subject invention are obtained from B.t. PS71M3, which has the following characteristics:

|  | *B.t.* PS71M3 |
|---|---|
| Colony morphology | large colony<br>dull surface<br>typical B.t. |
| Vegetative cell morphology | typical B.t. |
| Flagellar serotype | 8a8b, morrisoni |
| Intracellular inclusion | amorphic |
| Alkali-soluble proteins (SDS-PAGE) | 144,999<br>≈130,000<br>67,000<br>27,000 |

B.t. PS71M3 was obtained from B.t. PS71M3-69 (NRRL B-18515) as disclosed in Example 6.

The toxins encoded by the genes of the invention kill all mosquitoes tested. Bioassays have been done against Aedes aegypti, Aedes dorsalis, Anopheles albomanis, Culex pipiens quinquefasciatus, and Culex tarsalis.

Bioassay procedure: A dilution of toxin is added to water in a small cup. Fourth instar larvae are added. Mortality is read after 48 hours.

The cultures of the invention are as follows:

| Culture | Accession No. | Deposit date |
|---|---|---|
| Escherichia coli (NM522)(pMYC1625) | NRRL B-18644 | April 6, 1990 |
| Escherichia coli (NM522)(pMYC1636) | NRRL B-18693 | August 3, 1990 |
| Bacillus thuringiensis PS71M3-69 | NRRL B-18515 | June 29, 1989 |

The cultures were deposited in the Agricultural Research Service Patent Culture Collection (NRRL), Northern Regional Research Center, 1815 North University Street, Peoria, Illinois 61604 USA.

The toxin genes of the subject invention can be introduced into a wide variety of microbial hosts. Expression of the toxin genes results, directly or indirectly, in the intracellular production and maintenance of the pesticide. With suitable hosts, e.g., Pseudomonas, the microbes can be applied to the situs of dipteran insects where they will proliferate and be ingested by the insects. The result is a control of the unwanted insects. Alternatively, the microbe hosting the toxin gene(s) can be treated under conditions that prolong the activity of the toxin(s) produced in the cell. The treated cell then can be applied to the environment of target pest(s). The resulting product retains the toxicity of the B.t. toxin(s).

Where the B.t. toxin gene is introduced via a suitable vector into a microbial host, and said host is applied to the environment in a living state, it is essential that certain host microbes be used. Microorganism hosts are selected which are known to occupy the "phytosphere" (phylloplane, phyllosphere, rhizosphere, and/or rhizoplane) of one or more cops of interest. These microorganisms are selected so as to be capable of successfully competing in the particular environment (crop and other insect habitats) with the wild-type microorganisms, provide for stable maintenance and expression of the gene expressing the polypeptide pesticide, and, desirably, provide for improved protection of the pesticide from environmental degradation and inactivation.

A large number of microorganisms are known to inhabit the phylloplane (the surface of the plant leaves) and/or the rhizosphere (the soil surrounding plant roots) of a wide variety of important crops. These microorganisms include bacteria, algae, and fungi. Of particular interest are microorganisms, such as bacteria, e.g., genera Bacillus, Pseudomonas, Erwinia, Serratia, Klebsiella, Xanthomonas, Streptomyces, Rhizobium, Rhodopseudomonas, Methylophilius, Agrobacterium, Acetobacter, Lactobacillus, Arthrobacter, Azotobacter, Leuconostoc, and Alcaligenes; fungi, particularly yeast, e.g., genera Saccharomyces, Cryptococcus, Kluyveromyces, Sporobolomyces, Rhodotorula, Aureobasidium. Of particular interest are such phytosphere bacterial species as Pseudomonas Syringae, Pseudomonas fluorescens, Serratia marcescens, Acetobacter xylinum, Agrobacterium tumefaciens, Rhodopseudomonas spheroides, Xanthomonas campestris, Rhizobium melioti, Alcaligene entrophus, and Azotobacter vinlandii; and phytosphere yeast species such as Rhodotorula rubra, R. glutinis, R. marina, R. aurantiaca, Cryptococcus albidus, C. diffluens, C. laureentii, Saccharomyces rosei, S. pretoriensis, S. cerevisiae, Sporobolomyces roseum, S. odorus, Kluyveromyces Veronae, and Aureobasidium pollulans. Of particular interest are the pigmented microorganisms.

A wide variety of ways are available for introducing a B.t. gene expressing a toxin into the microorganism host under conditions which allow for stable maintenance and expression of the gene. One can provide for DNA constructs which include the transcriptional regulatory signals for expression of the toxin gene, the toxin gene under their regulatory control and a DNA sequence homologous with a sequence in the host organism, whereby integration will occur, and/or a replication system which is functional in the host, whereby integration or stable maintenance will occur.

The transcriptional initiation signals will include a promoter and a transcriptional initiation start site. In some instances, it may be desirable to provide for regulative expression of the toxin, where expression of the toxin will only occur after release into the environment. This can be achieved with operators or a region binding to an activator or enhancers, which are capable of induction upon a change in the physical or chemical environment of the microorganisms. For example, a temperature sensitive regulatory region may be employed, where the organisms may be grown up in the laboratory without expression of a toxin, but upon release into the environment, expression would begin. Other techniques may employ a specific nutrient medium in the laboratory, which inhibits the expression of the toxin, where the nutrient medium in the environment would allow for expression of the toxin. For translational initiation, a ribosomal binding site and an initiation codon will be present.

Various manipulations may be employed for enhancing the expression of the messenger RNA particularly by using an active promoter, as well as by employing sequences, which enhance the stability of the messenger RNA. The transcriptional and translational termination region will involve stop codon(s), a terminator region, and optionally, a polyadenylation signal. A hydrophobic "leader" sequence may be employed at the amino terminus of the translated polypeptide sequence in order to promote secretion of the protein across the inner membrane.

In the direction of transcription, namely in the 5' to 3' direction of the coding or sense sequence, the construct will involve the trancriptional regulatory region, if any, and the promoter, where the regulatory region may be either 5' or 3' of the promoter, the ribosomal binding site, the initiation codon, the structural gene having an open reading frame in phase with the initiation codon, the stop codon(s), the polyadenylation signal sequence, if any, and the terminator region. This sequence as a double strand may be used by itself for transformation of a microorganism host, but will usually be included with a DNA sequence involving a marker, where the second DNA sequence may be joined to the toxin expression construct during introduction of the DNA into the host.

By a marker is intended a structural gene which provides for selection of those hosts which have been modified or transformed. The marker will normally provide for selective advantage, for example, providing for biocide resistance, e.g., resistance to antibiotics or heavy metals; complementation, so as to provide prototropy to an auxotrophic host, or the like. Preferably, complementation is employed, so that the modified host may not only be selected, but may also be competitive in the field. One or more markers may be employed in the development of the constructs, as well as for modifying the host. The organisms may be further modified by providing for a competitive advantage against other wild-type microorganisms in the field. For example, genes expressing metal chelating agents, e.g., siderophores, may be introduced into the host along with the structural gene expressing the toxin. In this manner, the enhanced expression of a siderophore may provide for a competitive advantage for the toxin-producing host, so that it may effectively compete with the wild-type microorganisms and stably occupy a niche in the environment.

Where no functional replication system is present, the construct will also include a sequence of at least 50 basepairs (bp), preferably at least about 100 bp, and usually not more than about 1000 bp of a sequence homologous with a sequence in the host. In this way, the probability of legitimate recombination is enhanced, so that the gene will be integrated into the host and stably maintained by the host. Desirably, the toxin gene will be in close proximity to the gene providing for complementation as well as the gene providing for the competitive advantage. Therefore, in the event that a toxin gene is lost, the resulting organism will be likely to also lose the complementing gene and/or the gene providing for the competitive advantage, so that it will be unable to compete in the environment with the gene retaining the intact construct.

A large number of tanscriptional regulatory regions are available from a wide variety of microorganism hosts, such as bacteria, bacteriophage, cyanobacteria, algae, fingi, and the like. Various tanscriptional regulatory regions include the regions associated with the trp gene, lac gene, gal gene, the lambda left and right promoters, the Tac promoter, the naturally-occuring promoters associated with the toxin gene, where functional in the host. See for example, U.S. Patent Nos. 4,332,898, 4,342,832 and 4,356,270. The termination region may be the termination region normally associated with the transcriptional initiation region or a different transcriptional initiation region, so long as the two regions are compatible and functional in the host.

Where stable episomal maintenance or integration is desired, a plasmid will be employed which has a replication system which is functional in the host. The replication system may be derived from the chromosome, an episomal element normally present in the host or a different host, or a replication system from a virus which is stable in the lost. A large number of plasmid are available, such as pBR322, pACY184, RSF1010, pRO1614, and the like. See for example, Olson et al., (1982) J. Bacteriol. 150:6069, and Bagdasarian et al., (1981) Gene 16:237, and U.S. Patent Nos. 4,356,270, 4,362,817, and 4,371,625.

The B.t. gene can be introduced between the transcriptional and translational initiation region and the transcriptional and translational termination region, so as to be under the regulatory control of the initiation region. This construct will be included in a plasmid, which will include at least one replication system, but may include more than one, where one replication system is employed for cloning during the development of the plasmid and the second replication system is necessary for functioning in the ultimate host. In addition, one or more markers may be present, which have been described previously. Where integration is desired, the plasmid will desirably include a sequence homologous with the hos genome.

The transformants can be isolated in accordance with conventional ways, usually employing a selection technique, which allows for selection of the desired organisms as against unmodified organisms or transferring organism, when present. The transformants then can be tested for pesticidal activity.

Suitable host cells, where the pesticide-containing cells will be treated to prolong the activity of the toxin in the cell when the then trated cell is applied to the environment of target pest(s), may include either prokaryotes or eukaryotes, normally being limited to those cells which do not produce substances toxic to higher organisms, such as mammals. However, organisms which produce substances toxic to higher organisms could be used, where the toxin is unstable or the level of application sufficiently low as to avoid any possibility of toxicity to a mammalian host. As hosts, of particular interest will be the prokaryotes and the lower eukaryotes, such as fingi. Illustrative prokaryotes, both Gram-negative and -positive, include Enterobacteriaceae, such as Escherichia, Erwinia, Shigella, Salmonella, and Proteus: Bacillaceae; Rhizobiaceae, such as Rhizobium; Spirillaceae, such as photobacterium, Zymomonas, Serratia Aeromonas, Vibrio, Desulfovibrio, Spirillum; Lactobacillaceae; Pseudomonadaceae, such as Pseudomonas and Acetobacter; Azotobacteraceae, Actinomycetales, and Nitrobacteraceae. Among eukaryotes are fungi, such as Phycomycetes and Ascomycetes, which includes yeast, such as Saccharomyces and Schizosaccharomyces; and Basidiomycetes yeast, such as Rhodotorula, Aureobasidium, Sporobolomyces, and the like.

Characteristics of particular interest in selecting a host cell for purposes of production include ease of introducing the B.t. gene into the host, availability of expression systems, efficiency of expression, stability of the pesticide in the host, and the presence of auxiliary genetic capabilities. Characteristics of interest for use as a pesticide microcapsule include protective qualities for the pesticide, such as thick cell walls, pigmentation, and intracellular packaging or formation of inclusion bodies; leaf affinity; lack of mammalian toxicity; attractiveness to pests for ingestion; ease of killing and fixing without damage to the toxin; and the like. Other considerations include ease of formulation and handling, economics, storage stability, and the like.

Host organisms of particular interest include yeast, such as Rhodotorula sp., Aureobasidium sp., Saccharomyces sp., and Sporobolomyces sp.; phylloplane organisms such as Pseudomonas sp., Erwinia sp. and Flavobacterium sp.; or such other organisms as Escherichia, Lactobacillus sp., Bacillus sp., Streptomyces sp., and the like. Specific organisms include Pseudomonas aeruginosa, Pseudomonas fluorescens Saccharomyces cerevisiae, Bacillus thuringiensis, Escherichia coli, Bacillus subtilis, Streptomyces lividans and the like.

The cell will usually be intact and be substantially in the proliferative form when treated, rather than in a spore form, although in some instances spores may be employed.

Treatment of the microbial cell, e.g., a microbe containing the B.t. toxin gene, can be by chemical or physical means, or by a combination of chemical and/or physical means, so long as the technique does not deleteriously affect the properties of the toxin, nor diminish the cellular capability in protecting the toxin. Examples of chemical reagents are halogenating agents, particularly halogens of atomic no. 17-80. More particularly, iodine can be used under mild conditions and for sufficient time to achieve the desired results. Other suitable techniques include treatment with aldehydes, such as formaldehyde and glutaraldehyde; anti-infectives, such as zephiran chloride and cetylpyridinium chloride; alcohols, such as isopropyl and ethanol; various histologic fixatives, such as Lugol iodine, Bouin's fixative, and Helly's fixative (See: Humason, Gretchen L., Animal Tissue Techniques, W.H. Freeman and Company, 1967); or a combination of physical (heat) and chemical agents that preserve and prolong the activity of the toxin produced in the cell when the cell is administered to the host animal. Examples of physical means are short wavelenght radiation such as gamma-radiation and X-radiation, freezing, UV irradiation, lyophilization, and the like.

The cells generally will have enhanced structural stability which will enhance resistance to environmental conditions. Where the pesticide is in a proform, the method of inactivation should be selected so as not to inhibit processing of the proform to the mature form of the pesticide by the target pest pathogen. For example, formaldehyde will crosslink proteins and could inhibit processing of the proform of a polypeptide pesticide. The method of inactivation or killing retains at least a substantial portion of the bio-availability or bioactivity of the toxin.

The cellular hos containing the B.t. insecticidal gene may be grown in any convenient nutrient medium, where the DNA construct provide a selective advantage, providing for a selective medium so that substantially all or all of the cells retain the B.t. gene. These cells may then be harvested in accordance with conventional ways. Alternatively, the cells can be treated prior to harvesting.

The B.t. cells may be formulated in a variety of ways. They may be employed as wettable powders, granules or dust, by mixing with various inert materials, such as inorganic minerals (phyllosilicates, carbonates, sulfates, phosphates, and the like) or botanical materials (powdered corncobs, rice hulls, walnut shells, and the like). The formulations may include spreader-sticker adjuvants, stabilizing agents, other pesticidal additives, or surfactants. Liquid formulations may be aqueous-based or non-aqueous and employed as foams, gels, suspensions, emulsifiable concentrates, or the like. The ingredients may include rheological agents, surfactants, emulsifiers, dispersants, or polymers.

The pesticidal concentration will vary widely depending upon the nature of the particular formulation, particularly whether it is a concentrate or to be used directly. The pesticide will be present in at least 1% by weight and may be 100% by weight. The dry formulations will have from about 1-95% by weight of the pesticide while the liquid formulations will generally be from about 1-60% by weight of the solids in the liquid phase. The formulations will be generally have from about $10^2$ to about $10^4$ cells/mg. These formulations will be administered at about 50 mg (liquid or dry) to 1 kg or more per hectare.

The formulations can be applied to the environment of the lepidopteran pest(s), e.g., plants, soil or water, by spraying, dusting, spinkling, or the like.

Mutants of B.t. PS71M3 can be made by procedures well known in the art. For example, an asporogenous mutant can be obained throught ethylmethane sulfonate (EMS) mutagenesis of B.t. PS71M3. The mutants can be made using ultraviolet light and nitrosoguanidine by procedures well known in the art.

A smaller percentage of the asporogenous mutants will remain intact and not lyse for extended fermentation periods; these strains are designated lysis minus (—). Lysis minus strains can be identified by screening asporogenous mutants in shake flask media and selecting those muatnts that are still intact and contain toxin crystals at the end of the fermentation. Lysis minus strains are suitable for a cell fixation process that will yield a protected, encapsulated toxin protein.

To prepare a phage resistant variant of said asporogenous mutant, an aliquot of the phage lysate is spread onto nutrient agar and allowed to dry. An aliquot of the phage sensitive bacterial strain is then plated directly over the dried lysate and allowed to dry. The plates are incubated at 30°C. The plates are incubated for 2 days and, at that time, numerous colonies could be seen growing on the agar. Some of these colonies are picked

5

and subcultured onto nutrient agar plates. These apparent resistant cultures are tested for resistance by cross streaking with the phage lysate. A line of the phage lysate is streaked on the plate and allowed to dry. The presumptive resistant cultures are then streaked across the phage line. Resistant bacterial cultures show no lysis anywhere in the streak across the phage line after overnight incubation at 30°C. The resistance to phage is then reconfirmed by plating a lawn of the resistant culture onto a nutrient agar plate. The sensitive strain is also plated in the same manner to serve as the positive control. After drying, a drop of the phage lysate is plated in the center of the plate and allowed to dry. Resistant cultures showed no lysis in the area where the phage lysate has been placed after incubation at 30°C for 24 hours.

Following are examples which illustrate procedures, including the best mode, for practicing the invention. These examples should not be construed as limiting. All percentages are by weight and all solvent mixture proportions are by volume unless otherwise noted.

Example 1 - Culturing _B.t._ PS71M3

A subculture of B.t. PS71M3, or mutants thereof, can be used to inoculate the following medium, a peptone, glucose, salts medium.

| | |
|---|---|
| Bacto Peptone | 7.5 g/l |
| Glucose | 1.0 g/l |
| $KH_2PO_4$ | 3.4 g/l |
| $K_2HPO_4$ | 4.35 g/l |
| Salt Solution | 5.0 ml/l |
| $CaCl_2$ Solution | 5.0 ml/l |

Salts Solution (100 ml)

$MgSO_4.7H_2O$ 2.46 g
$MnSO_4.H_2O$ 0.04 g
$ZnSO_4.7H_2O$ 0.28 g
$FeSO_4.7H_2O$ 0.40 g

$$CaCl_2 \text{ Solution (100 ml)}$$

$$CaCl_2.2H_2O \qquad\qquad 3.66 \text{ g}$$

$$pH\ 7.2$$

The salts solution and $CaCl_2$ solution are filter-sterilized and added to the autoclaved and cooked broth at the time of inoculation. Flasks are incubated at 30°C on a rotary shaker at 200 rpm for 64 hr.

The above procedure can be readily scaled up to large fermentors by procedures well known in the art.

The B.t. spores and/or crystals, obtained in the above fermentation, can be isolated by procedures well known in the art. A frequently-used procedure is to subject the harvested fermentation broth to separation techniques, e.g., centrifugation.

Example 2 - Cloning of ≈ 130 kD Novel Toxin Gene from Isolate _B.t._ PS71M3 and Transformation into _Escherichia coli_

Total cellular DNA was prepared from B.t. cells grown to a low optical density ($OD_{600}$ = 1.0). The cells were recovered by centrifugation and protoplasted in TES buffer (30 mM Tris-Cl, 10 mM ethylenediaminetetraacetic acid [EDTA], 50 mM NaCl, pH = 8.0) containing 20% sucrose and 50 mg/ml lysozyme. The protoplasts were lysed by addition of sodium dodecyl sulfate (SDS) to a final concentration of 4%. The cellular material was precipitated overnight at 4°C in 100 mM (final concentration) neutral potassium chloride. The supernate was extracted twice with phenol/chloroform (1:1). The DNA was precipitated with ethanol and purified by isopycnic banding on a cesium gradient.

Total cellular DNA from B.t. PS71M3 was digested with EcoRV and separated by electrophoresis on a 0.8% (w/v) Agarose-TAE (50 mM Tris-Cl 20 mM NaOAc, 2.5 mM EDTA pH=8.0) buffered gel. A Southern blot of the gel was hybridized with a [$^{32}$P] radiolabeled probe. The sequence of the oligonucleotide is (GGTGATTTTACA-CAAGGGGTAATGGGGTGGCATG). Results showed that the hybridizing fragments of B.t. PS71M3 are 4.8 kb, 4.0 kb, and 3.8 kb in size.

A library was constructed from B.t. PS71M3 total cellular DNA partially digested with Sau3A and size fractionated by electrophoresis. The 9 to 23 kb region of the gel was excised and the DNA was electroeluted and then concentrated using an Elutip™ ion exchange column (Scleicher an Schuel, Keene, NH). The isolated Sau3A fragments were ligated into LambdaGEM-11™ (PROMEGA). The packaged phage were plated on KW251 E. coli cells (PROMEGA) at a high titer and screened using the radiolabeled synthetic oligonucleotide as a nucleic acid hybridization probe. Hybridizing plaques were purified and rescreened at a lower plaque density. Single isolated purified plaques that hybridized with the probe were used to infect KW251 E. coli cells in liquid culture for preparation of phage for DNA isolation. DNA was isolated by standard procedures. Preparative amounts of DNA were digested with SalI (to release the inserted DNA from lambda arms) and separated by electrophoresis on a 0.6% agarose-TAE gel. The large fragments, electroeluted and concentrated as described above, were ligated to SalI-digested and dephosphorylated pBClac. The ligation mix was introduced by transformation into NM522 competent E. coli cells and plated on LB agar containing ampicillin, isopropyl-(Beta)-D-thiogalactoside (IPTG) and 5-bromo-4chloro-3-indolyl-(Beta)-D-galactoside (XGAL). White colonies, with putative insertions in the (Beta)-galactosidase gene of pBClac, were subjected to standard rapid plasmid purification procedures to isolate the desired plasmid. The selected plasmid was named pMYC1625 and contains an 8.0 kb SalI insert.

The restriction map of the cloned insert indicates that the toxin gene is novel compared to the maps of other toxin genes encoding mosquitocidal proteins. The restriction map of the toxin gene cloned in pMYC1625, is in the family of the cryIVA genes (Höfte, H., and H.R. Whiteley [1989] Microbiological Reviews 53:242-255); the 140 kD endotoxin gene (Ward, S., and D. Ellar [1987] Nucleic Acids Res. 15:7195), and the type II gene (Sen et al. [1988] Agric. Biol. Chem. 52:873-878) isolated from Bacillus thuringiensis var. israelensis (B.t.i.).

The toxin gene was sequenced by the standard Sanger dideoxy chain termination method using oligonucleotide primers made to the B.t.i. cryIVA gene and by "walking" with primers made to the sequence of the new toxin gene. Sequence analysis of the toxin gene revealed that it encodes a protein of 134,934 daltons, deduced from the DNA sequence. The nucleotide sequence and deduced amino acid sequence are given below as Seq. ID No.1.

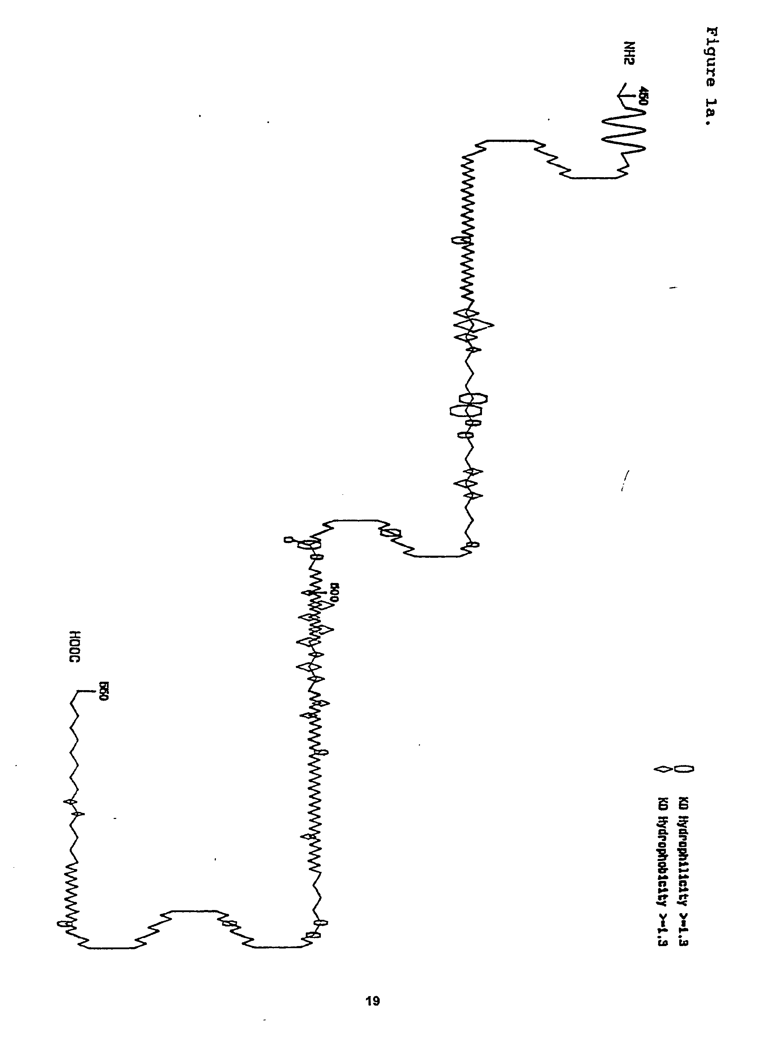

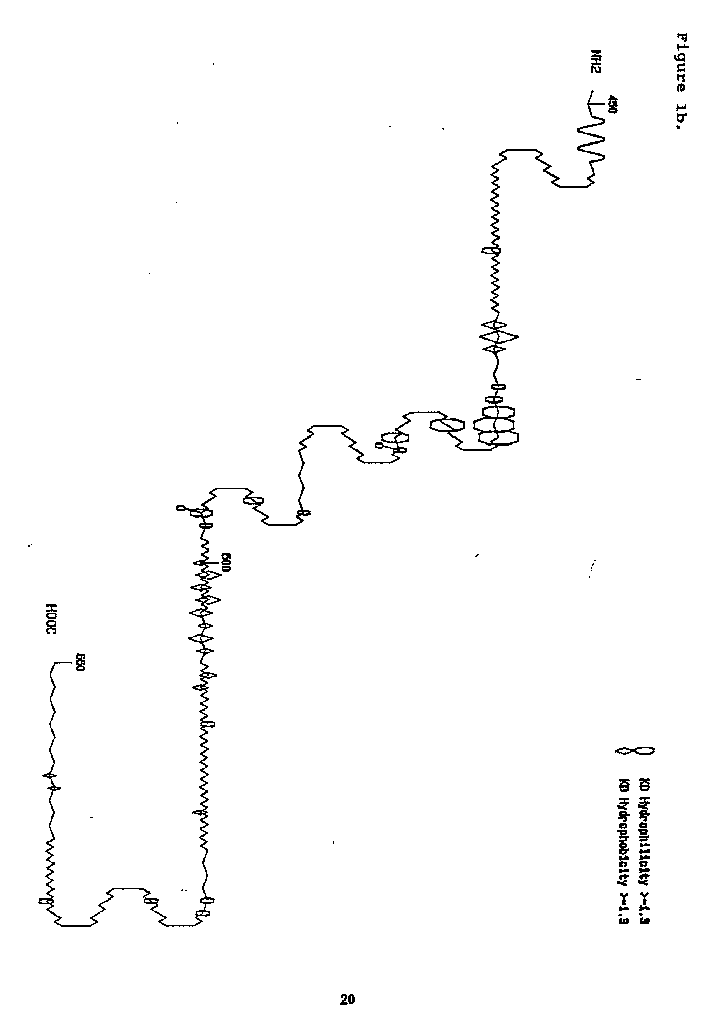

Six predicted amino acid differences between the type II gene, referred to above, and the subject protein, are believed to be significant determinants with respect to the secondary structure of the expressed gene product, as analyzed by the method of Chou and Fasman ([1978] Adv. in Enzymol. 47:45-148). While the changes that occur in the amino acid sequences of the two protein gene expression products are considered to be conservative in nature at amino acid numbers 486, 796, and 886, those occurring at amino acid numbers 481, 487, and 987 introduce different functionalities which in turn can impart profound changes in the predicted secondary structure of the protein. This is illustrated in Figures 1a and 1b. Whereas the subject gene product has a single predicted turn between amino acids 470 and 500, the type II protein contains 3 turns within the same region.

Plasmid pMYC1625 was introduced into a cured, acrystalliferous (cry⁻) B.t. host by electroporation. Expression of an ≈ 130 kD protein was verified by SDS-PAGE. Spores and crystals were used for the determination of toxicity to mosquito larvae. $10^{-4}$ ml of culture/ml of solution resulted in 100% mortality of Aedes aegypti and Culex pipiens after a 24 hour period.

The various methods employed in the preparation of the plasmids and transformation of host organisms are well known in the art. See Maniatis, T., E.F. Fritsch, and J. Sambrook (1982) Molecular Cloning: A Laboratory Manual, Cold Spring Harbor Laboratory, New York.

The restriction enzymes disclosed herein can be purchased from Bethesda Research Laboratories, Gaithersburg, MD; New England Biolabs, Beverly, MA; or Boehringer-Mannheim, Indianapolis, IN. The enzymes are used according to the instructions provided by the supplier.

The pBClac shuttle vector was constructed by fusing plasmids pBC16-1 (Bacillius Genetic Stock Center, Ohio State University, Department of Biochemistry, Columbus, OH) and pUC19 (New England Biolabs). The pBC16-1 plasmid was digested with EcoRI and the 5′ overhangs were filled in with deoxynucleotides (dATP, dCTP, dGTP, and dTTP) by Klenow enzyme (New England Biolabs). An SpeI restriction site was added by ligation of oligonucleotide linkers forming pBC16-1SpeI. In the same manner as above, an NheI restriction site was added to pUC19 at the Eco0109 site forming pUC19NheI. The pBC16-1SpeI plasmid was digested with SpeI and the pUC19NheI plasmid was digested with NheI, creating complementary cohesive ends that were ligated together to form the pBClac shuttle vector.

The plasmid containing the B.t. toxin gene can be removed from the transformed host microbe by use of standard well-known procedures. For example, the host microbe can be subjected to cleared lysate isopycnic density gradient procedures, and the like, to recover the desised plasmid.

The above gene can also be isolated from B.t. PS71M3-69 by the same procedures.

## Example 3 - Cloning of ≈ 77 kD Novel Toxin Gene From Isolate *B.t.* PS71M3 and Transformation into *Escherichia coli*

As described in Example 2, total cellular DNA was prepared. Total cellular DNA from B.t. PS71M3 was digested with EcoRV and separated by electrophoresis on a 0.8% (w/v) agarose-TAE (50 mM Tris-HCl, 20 mM NaOAc, 2.5 mM EDTA, ph=8.0) buffered gel. A Southern blot of the gel was hybridized with a [$^{32}$P]-radiolabeled oligonucleotide probe. The sequence of the oligonucleotide is CCAAGGGCGTTTTTACACAAGAAATTCTC-GAGAC. Results showed that the hybridizing fragments of B.t. PS71M3 are approximately 14.0 kb and 2.9 kb in size.

A library was constructed as described in Example 2. White colonies, with putative insertions in the (β)-galactosidase gene of pBClac, were subjected to standard rapid plasmid purification procedures to isolate the desired plasmid. The selected plasmid was named pMYC1636 and contains an approximately 15 kb SalI insert.

The restriction map of the cloned insert indicates that the toxin gene is novel compared to the maps of other toxin genes encoding mosquitocidal proteins. The restriction map of the toxin gene cloned in pMYC1636 is related to the cryIVC gene (Höfte and Whiteley, supra) isolated from B.t. var. israelensis (B.t.i.).

The toxin gene was sequenced by the standard dideoxy chain termination method using oligonucleotide primers made to the B.t.i. cryIVC gene and by "walking" with primers made to the sequence of the new toxin gene. Sequence analysis of the toxin gene revealed an open reading frame that is ≈95% homologous to the cryIVC gene, and encodes a protein of 77,798 daltons, deduced from the DNA sequence, that has 2 different amino acids. The nucleotide sequence and deduced amino acid sequence are given below as Seq. ID No. 2.

Plasmid pMYC1636 was introduced into an acrystalliferous (cry-) B.t. host by electroporation. Expression of an approximately 68 kD processed protein was verified by SDS-PAGE. Spores and crystals were used for the determination of toxicity to mosquito larvae. 10$^{-4}$ ml of culture/ml of solution resulted in 100% mortality of Aedes aegypti after a 24 hour period.

The above gene also can be isolated from B.t. PS71M3-69 by the same procedures.

## Example 4 - Insertion of Toxin Genes Into Plants

The novel genes coding for the novel insecticidal toxins, as disclosed herein, can be inserted in plant cells using the Ti plasmid from Agrobacter tumefaciens. Plant cells can then be caused to regenerate into plants (Zambryski, P., H. Joos, C. Gentello, J. Leemans, M. Van Montague, and J. Schell [1983] Cell 32:1033-1043). A particularly useful vector in this regard is pEND4K (Klee, H.J., M.F. Yanofsky, and E.W. Nester [1985] Bio/Technology 3:637-642). This plasmid can replicate both in plant cells and in bacteria and has multiple cloning sites for passenger genes. The toxin gene, for example, can be inserted into the BamHI site of pEND4K, propagated in E. coli, and transformed into appropriate plant cells.

## Example 5 - Cloning of Novel *Bacillus thuringiensis* Genes Into Baculoviruses

The novel genes of the invention can be cloned into baculoviruses such as Autographa californica nuclear polyhedrosis virus (AcNPV). Plasmids can be constructed that contain the AcNPV genome cloned into a commercial cloning vector such as pUC8. The AcNPV genoine is modified so that the coding region of the polyhedrin gene is removed and a unique cloning site for a passenger gene is placed directly behind the polyhedrin promoter. Examples of such vectors are pGP-B6874, described by Pennock et al. (Pennock, G.D., C. Shoemaker, and L.K. Miller [1984] Mol. cell. Biol. 4:399-406), and pAC380, described by Smith et al. (Smith, G.E., M.D. Summers, and M.J. Fraser [1983] Mol. cell. Biol. 3:2156-2165). The genes coding for the novel protein toxins of the invention can be modified with BamHI linkers at appropriate regions both upstream and downstream from the coding region and inserted into the passenger site of one of the AcNPV vectors.

## Example 6 - Preparation of Parent Strain *B.t.* PS71M3 From *B.t.* PS71M3-69

The parent strain can be obtained under stressed conditions, such as pasteurization. Under the conditions that are described below, the following reversion frequency has been established:

$$PS71M3-69 \qquad 1.63 \times 10^{-11}$$

Statistically, this indicates that one out of $1 \times 10^{11}$ cells will be a revertant. Gel scan analysis of the proteins produced upon outgrowth of the spore-formers (revertants) found after pasteurization was identical to the parents for both strains.

The procedure is as follows:

1. Grow mutant culture (B.t. PS71M3-69) in 1 liter of dilute medium (L-Broth) for 24 hours on a rotary shaker.

2. Harvest cell suspension and transfer to sterile centrifuge bottles and spin 10 minutes at 8,000 rpm.

3. Remove the supernatant and re-suspend the pellets in enough sterile deionized water to bring the volume to 50 ml.

4. Re-suspend the pellets and transfer to a sterile flask. Prepare a plate count from this untreated material.

5. Place the flask in an 80°C water bath, shake at 200 rpm for 15 minutes.

6. Remove the flask and plate the suspension on Nutrient agar (50 plates for 50 mls).

7. Incubate the plates for 48 hours at 30°C and microscopically examine any colonies that appear.

8. Subculture any spore-formers onto Nutrient agar and incubate for 72 hours. The reversion frequency is calculated by dividing the number of spore-formers by the total number of cells treated.

Example: $(8.9 \times 10^9 \text{ cfu/ml}) \times 50 \text{ ml} = 4.45 \times 10^{11}$ cells treated

3 spore-formers found

$3/4.45 \times 10^{11} = 6.74 \times 10^{-12}$

9. To determine the identity of the spore-formers, grow the "revertants" in liquid fermentation medium parallel to the parent strain.

10. Analyse and compare the proteins produced to those produced by the parent. The "revertants" and the parent strains should have identical banding patterns.

It is well known in the art that the amino-acid sequence of a protein is determined by the nucleotide sequence of the DNA. Because of the redundancy of the genetic code, i.e. more than one coding nucleotide triplet (codon) can be used for most of the amino-acids used to make proteins, different nucleotide sequences can code for a particular amino-acid.

The novel B.t. toxins can be prepared via any nucleotide sequence (equivalent to that shown) encoding the same amino-acid sequence; the present invention includes such equivalent nucleotide sequences.

It has been shown that proteins of identified structure and function may be constructed by changing the amino-acid sequence, if such changes do not alter the protein secondary structure; see Kaiser, E.T. and Kezdy, F.J. (1984) Science 223:249-255. The present invention includes mutants of the amino-acid sequences depicted herein which have an unaltered protein secondary structure or, if the structure is altered, the mutant has the biological activity retained to some degree.

## SEQUENCE LISTING

SEQ ID No. 1
SEQUENCE TYPE: Nucleotide with corresponding protein
SEQUENCE LENGTH: 3543 base pairs
STRANDEDNESS: single
TOPOLOGY: linear
MOLECULE TYPE: genomic DNA
ORIGINAL SOURCE ORGANISM: Bacillus thuringiensis var. morrisoni (PS71M3)
PROPERTIES: Dipteran-active toxin gene

```
              5                    10                   15                   20
Met Asn Pro Tyr Gln Asn Lys Asn Glu Tyr Glu Thr Leu Asn Ala Ser Gln Lys Lys Leu
ATG AAT CCT TAT CAA AAT AAA AAT GAA TAT GAA ACA TTA AAT GCT TCA CAA AAA AAA TTA


              25                   30                   35                   40
Asn Ile Ser Asn Asn Tyr Thr Arg Tyr Pro Ile Glu Asn Ser Pro Lys Gln Leu Leu Gln
AAT ATA TCT AAT AAT TAT ACA AGA TAT CCA ATA GAA AAT AGT CCA AAA CAA TTA TTA CAA


              45                   50                   55                   60
Ser Thr Asn Tyr Lys Asp Trp Leu Asn Met Cys Gln Gln Asn Gln Gln Tyr Gly Gly Asp
AGT ACA AAT TAT AAA GAT TGG CTC AAT ATG TGT CAA CAG AAT CAG CAG TAT GGT GGA GAT


              65                   70                   75                   80
Phe Glu Thr Phe Ile Asp Ser Gly Glu Leu Ser Ala Tyr Thr Ile Val Val Gly Thr Val
TTT GAA ACT TTT ATT GAT AGT GGT GAA CTC AGT GCC TAT ACT ATT GTA GTT GGG ACC GTA


              85                   90                   95                   100
Leu Thr Gly Phe Gly Phe Thr Thr Pro Leu Gly Leu Ala Leu Ile Gly Phe Gly Thr Leu
CTG ACT GGT TTC GGG TTC ACA ACA CCC TTA GGA CTT GCT TTA ATA GGT TTT GGT ACA TTA


              105                  110                  115                  120
Ile Pro Val Leu Phe Pro Ala Gln Asp Gln Ser Asn Thr Trp Ser Asp Phe Ile Thr Gln
ATA CCA GTT CTT TTT CCA GCC CAA GAC CAA TCT AAC ACA TGG AGT GAC TTT ATA ACA CAA


              125                  130                  135                  140
Thr Lys Asn Ile Ile Lys Lys Glu Ile Ala Ser Thr Tyr Ile Ser Asn Ala Asn Lys Ile
ACT AAA AAT ATT ATA AAA AAA GAA ATA GCA TCA ACA TAT ATA AGT AAT GCT AAT AAA ATT


              145                  150                  155                  160
Leu Asn Arg Ser Phe Asn Val Ile Ser Thr Tyr His Asn His Leu Lys Thr Trp Glu Asn
TTA AAC AGG TCG TTT AAT GTT ATC AGC ACT TAT CAT AAT CAC CTT AAA ACA TGG GAG AAT


              165                  170                  175                  180
Asn Pro Asn Pro Gln Asn Thr Gln Asp Val Arg Thr Gln Ile Gln Leu Val His Tyr His
AAT CCA AAC CCA CAA AAT ACT CAG GAT GTA AGG ACA CAA ATC CAG CTA GTT CAT TAC CAT


              185                  190                  195                  200
Phe Gln Asn Val Ile Pro Glu Leu Val Asn Ser Cys Pro Pro Asn Pro Ser Asp Cys Asp
TTT CAA AAT GTC ATT CCA GAG CTT GTA AAC TCT TGT CCT CCT AAT CCT AGT GAT TGC GAT


              205                  210                  215                  220
Tyr Tyr Asn Ile Leu Val Leu Ser Ser Tyr Ala Gln Ala Ala Asn Leu His Leu Thr Val
TAC TAT AAC ATA CTA GTA TTA TCT AGT TAT GCA CAA GCA GCA AAC TTA CAT CTG ACT GTA


              225                  230                  235                  240
Leu Asn Gln Ala Val Lys Phe Glu Ala Tyr Leu Lys Asn Asn Arg Gln Phe Asp Tyr Leu
TTA AAT CAA GCC GTC AAA TTT GAA GCG TAT TTA AAA AAC AAT CGA CAA TTC GAT TAT TTA


              245                  250                  255                  260
Glu Pro Leu Pro Thr Ala Ile Asp Tyr Tyr Pro Val Leu Thr Lys Ala Ile Glu Asp Tyr
GAG CCT TTG CCA ACA GCA ATT GAT TAT TAT CCA GTA TTG ACT AAA GCT ATA GAA GAT TAC


              265                  270                  275                  280
Thr Asn Tyr Cys Val Thr Thr Tyr Lys Lys Gly Leu Asn Leu Ile Lys Thr Thr Pro Asp
ACT AAT TAT TGT GTA ACA ACT TAT AAA AAA GGA TTA AAT TTA ATT AAA ACG ACG CCT GAT
```

                    285                 290                 295                 300
Ser Asn Leu Asp Gly Asn Ile Asn Trp Asn Thr Tyr Asn Thr Tyr Arg Thr Lys Met Thr
AGT AAT CTT GAT GGA AAT ATA AAC TGG AAC ACA TAC AAT ACG TAT CGA ACA AAA ATG ACT

                    305                 310                 315                 320
Thr Ala Val Leu Asp Leu Val Ala Leu Phe Pro Asn Tyr Asp Val Gly Lys Tyr Pro Ile
ACT GCT GTA TTA GAT CTT GTT GCA CTC TTT CCT AAT TAT GAT GTA GGT AAA TAT CCA ATA

                    325                 330                 335                 340
Gly Val Gln Ser Glu Leu Thr Arg Glu Ile Tyr Gln Val Leu Asn Phe Glu Glu Ser Pro
GGT GTC CAA TCT GAA CTT ACT CGA GAA ATT TAT CAG GTA CTT AAC TTC GAA GAA AGC CCC

                    345                 350                 355                 360
Tyr Lys Tyr Tyr Asp Phe Gln Tyr Gln Glu Asp Ser Leu Thr Arg Arg Pro His Leu Phe
TAT AAA TAT TAT GAC TTT CAA TAT CAA GAG GAT TCA CTT ACA CGT AGA CCG CAT TTA TTT

                    365                 370                 375                 380
Thr Trp Leu Asp Ser Leu Asn Phe Tyr Glu Lys Ala Gln Thr Thr Pro Asn Asn Phe Phe
ACT TGG CTT GAT TCT TTG AAT TTT TAT GAA AAA GCG CAA ACT ACT CCT AAT AAT TTT TTC

                    385                 390                 395                 400
Thr Ser His Tyr Asn Met Phe His Tyr Thr Leu Asp Asn Ile Ser Gln Lys Ser Ser Val
ACC AGC CAT TAT AAT ATG TTT CAT TAC ACA CTT GAT AAT ATA TCC CAA AAA TCT AGT GTT

                    405                 410                 415                 420
Phe Gly Asn His Asn Val Thr Asp Lys Leu Lys Ser Leu Gly Leu Ala Thr Asn Ile Tyr
TTT GGA AAT CAC AAT GTA ACT GAT AAA TTA AAA TCT CTT GGT TTG GCA ACA AAT ATT TAT

                    425                 430                 435                 440
Ile Phe Leu Leu Asn Val Ile Ser Leu Asp Asn Lys Tyr Leu Asn Asp Tyr Asn Asn Ile
ATT TTT TTA TTA AAT GTC ATA AGC TTA GAT AAT AAA TAT CTA AAT GAT TAT AAT AAT ATT

                    445                 450                 455                 460
Ser Lys Met Asp Phe Phe Ile Thr Asn Gly Thr Arg Leu Leu Glu Lys Glu Leu Thr Ala
AGT AAA ATG GAT TTT TTT ATA ACT AAT GGT ACT AGA CTT TTG GAG AAA GAA CTT ACA GCA

                    465                 470                 475                 480
Gly Ser Gly Gln Ile Thr Tyr Asp Val Asn Lys Asn Ile Phe Gly Leu Pro Ile Leu Lys
GGA TCT GGG CAA ATA ACT TAT GAT GTA AAT AAA AAT ATT TTC GGG TTA CCA ATT CTT AAA

                    485                 490                 495                 500
Pro Arg Glu Asn Gln Ala Ile Pro Thr Leu Phe Pro Thr Tyr Asp Asn Tyr Ser His Ile
CCA AGA GAG AAT CAA GCA ATC CCT ACC CTT TTT CCA ACA TAT GAT AAC TAT AGT CAT ATT

                    505                 510                 515                 520
Leu Ser Phe Ile Lys Ser Leu Ser Ile Pro Ala Thr Tyr Lys Thr Gln Val Tyr Thr Phe
TTA TCA TTT ATT AAA AGT CTT AGT ATC CCT GCA ACA TAT AAA ACT CAA GTG TAT ACG TTT

                    525                 530                 535                 540
Ala Trp Thr His Ser Ser Val Asp Pro Lys Asn Thr Ile Tyr Thr His Leu Thr Thr Gln
GCT TGG ACA CAC TCT AGT GTT GAT CCT AAA AAT ACA ATT TAT ACA CAT TTA ACT ACC CAA

                    545                 550                 555                 560
Ile Pro Ala Val Lys Ala Asn Ser Leu Gly Thr Ala Ser Lys Val Val Gln Gly Pro Gly
ATT CCA GCT GTA AAA GCG AAT TCA CTT GGG ACT GCT TCT AAG GTT GTT CAA GGA CCT GGT

                    565                 570                 575                 580
His Thr Gly Gly Asp Leu Ile Asp Phe Lys Asp His Phe Lys Ile Thr Cys Gln His Ser
CAT ACA GGA GGG GAT TTA ATT GAT TTC AAA GAT CAT TTC AAA ATT ACA TGT CAA CAC TCA

11

EP 0 457 498 A2

```
                585              590              595              600
      Asn Phe Gln Gln Ser Tyr Phe Ile Arg Ile Arg Tyr Ala Ser Asn Gly Ser Ala Asn Thr
      AAT TTT CAA CAA TCG TAT TTT ATA AGA ATT CGT TAT GCT TCA AAT GGA AGC GCA AAT ACA

                605              610              615              620
      Arg Ala Val Ile Asn Leu Ser Ile Pro Gly Val Ala Glu Leu Gly Met Ala Leu Asn Pro
      CGA GCT GTT ATA AAT CTT AGT ATC CCA GGG GTA GCA GAA CTG GGT ATG GCA CTC AAC CCC

                625              630              635              640
      Thr Phe Ser Gly Thr Asp Tyr Thr Asn Leu Lys Tyr Lys Asp Phe Gln Tyr Leu Glu Phe
      ACT TTT TCT GGT ACA GAT TAT ACG AAT TTA AAA TAT AAA GAT TTT CAG TAC TTA GAA TTT

                645              650              655              660
      Ser Asn Glu Val Lys Phe Ala Pro Asn Gln Asn Ile Ser Leu Val Phe Asn Arg Ser Asp
      TCT AAC GAG GTG AAA TTT GCT CCA AAT CAA AAC ATA TCT CTT GTG TTT AAT CGT TCG GAT

                665              670              675              680
      Val Tyr Thr Asn Thr Thr Val Leu Ile Asp Lys Ile Glu Phe Leu Pro Ile Thr Arg Ser
      GTA TAT ACA AAC ACA ACA GTA CTT ATT GAT AAA ATT GAA TTT CTG CCA ATT ACT CGT TCT

                685              690              695              700
      Ile Arg Glu Asp Arg Glu Lys Gln Lys Leu Glu Thr Val Gln Gln Ile Ile Asn Thr Phe
      ATA AGA GAG GAT AGA GAG AAA CAA AAA TTA GAA ACA GTA CAA CAA ATA ATT AAT ACA TTT

                705              710              715              720
      Tyr Ala Asn Pro Ile Lys Asn Thr Leu Gln Ser Glu Leu Thr Asp Tyr Asp Ile Asp Gln
      TAT GCA AAT CCT ATA AAA AAC ACT TTA CAA TCA GAA CTT ACA GAT TAT GAC ATA GAT CAA

                725              730              735              740
      Ala Ala Asn Leu Val Glu Cys Ile Ser Glu Glu Leu Tyr Pro Lys Glu Lys Met Leu Leu
      GCC GCA AAT CTT GTG GAA TGT ATT TCT GAA GAA TTA TAT CCA AAA GAA AAA ATG CTG TTA

                745              750              755              760
      Leu Asp Glu Val Lys Asn Ala Lys Gln Leu Ser Gln Ser Arg Asn Val Leu Gln Asn Gly
      TTA GAT GAA GTT AAA AAT GCG AAA CAA CTT AGT CAA TCT CGA AAT GTA CTT CAA AAC GGG

                765              770              775              780
      Asp Phe Glu Ser Ala Thr Leu Gly Trp Thr Thr Ser Asp Asn Ile Thr Ile Gln Glu Asp
      GAT TTT GAA TCG GCT ACG CTT GGT TGG ACA ACA AGT GAT AAT ATC ACA ATT CAA GAA GAT

                785              790              795              800
      Asp Pro Ile Phe Lys Gly His Tyr Leu His Met Ser Gly Ala Arg Glu Ile Asp Gly Thr
      GAT CCT ATT TTT AAA GGG CAT TAC CTT CAT ATG TCT GGG GCG AGA GAA ATT GAT GGT ACG

                805              810              815              820
      Ile Phe Pro Thr Tyr Ile Phe Gln Lys Ile Asp Glu Ser Lys Leu Lys Pro Tyr Thr Arg
      ATA TTT CCG ACC TAT ATA TTC CAA AAA ATT GAT GAA TCA AAA TTA AAA CCG TAT ACA CGT

                825              830              835              840
      Tyr Leu Val Arg Gly Phe Val Gly Ser Ser Lys Asp Val Glu Leu Val Val Ser Arg Tyr
      TAC CTA GTA AGG GGA TTT GTA GGA AGT AGT AAA GAT GTA GAA CTA GTG GTT TCA CGC TAT

                845              850              855              860
      Gly Glu Glu Ile Asp Ala Ile Met Asn Val Pro Ala Asp Leu Asn Tyr Leu Tyr Pro Ser
      GGG GAA GAA ATT GAT GCC ATC ATG AAT GTT CCA GCT GAT TTA AAC TAT CTG TAT CCT TCT

                865              870              875              880
      Thr Phe Asp Cys Glu Gly Ser Asn Arg Cys Glu Thr Ser Ala Val Pro Ala Asn Ile Gly
      ACC TTT GAT TGT GAA GGG TCT AAT CGT TGT GAG ACG TCC GCT GTG CCG GCT AAC ATT GGG
```

12

```
              885              890              895              900                    ***
Asn Thr Ser Asp Met Ser Tyr Ser Cys Gln Tyr Asp Thr Gly Lys Lys His Val Val Cys        TGA
AAC ACT TCT GAT ATG TCG TAT TCA TGC CAA TAT GAT ACA GGG AAA AAG CAT GTC GTA TGT

              905              910              915              920
Gln Asp Ser His Gln Phe Ser Phe Thr Ile Asp Thr Gly Ala Leu Asp Thr Asn Glu Asn
CAG GAT TCC CAT CAA TTT AGT TTC ACT ATT GAT ACA GGG GCA TTA GAT ACA AAT GAA AAT

              925              930              935              940
Ile Gly Val Trp Val Met Phe Lys Ile Ser Ser Pro Asp Gly Tyr Ala Ser Leu Asp Asn
ATA GGG GTT TGG GTC ATG TTT AAA ATA TCT TCT CCA GAT GGA TAC GCA TCA TTA GAT AAT

              945              950              955              960
Leu Glu Val Ile Glu Glu Gly Pro Ile Asp Gly Glu Ala Leu Ser Arg Val Lys His Met
TTA GAA GTA ATT GAA GAA GGG CCA ATA GAT GGG GAA GCA CTG TCA CGC GTG AAA CAC ATG

              965              970              975              980
Glu Lys Lys Trp Asn Asp Gln Met Glu Ala Lys Arg Ser Glu Thr Gln Gln Ala Tyr Asp
GAG AAG AAA TGG AAC GAT CAA ATG GAA GCA AAA CGT TCG GAA ACA CAA CAA GCA TAT GAT

              985              990              995              1000
Val Ala Lys Gln Ala Ile Asn Ala Leu Phe Thr Asn Val Gln Asp Glu Ala Leu Gln Phe
GTA GCG AAA CAA GCC ATT AAT GCT TTA TTC ACA AAT GTA CAA GAT GAG GCT TTA CAG TTT

              1005             1010             1015             1020
Asp Thr Thr Leu Ala Gln Ile Gln Tyr Ala Glu Tyr Leu Val Gln Ser Ile Pro Tyr Val
GAT ACG ACA CTC GCT CAA ATT CAG TAC GCT GAG TAT TTG GTA CAA TCG ATT CCA TAT GTG

              1025             1030             1035             1040
Tyr Asn Asp Trp Leu Ser Asp Val Pro Gly Met Asn Tyr Asp Ile Tyr Val Glu Leu Asp
TAC AAT GAT TGG TTG TCA GAT GTT CCA GGT ATG AAT TAT GAC ATC TAT GTA GAG TTG GAT

              1045             1050             1055             1060
Ala Arg Val Ala Gln Ala Arg Tyr Leu Tyr Asp Thr Arg Asn Ile Ile Lys Asn Gly Asp
GCA CGA GTG GCA CAA GCG CGT TAT TTG TAT GAT ACA AGA AAT ATT ATT AAA AAT GGT GAT

              1065             1070             1075             1080
Phe Thr Gln Gly Val Met Gly Trp His Val Thr Gly Asn Ala Asp Val Gln Gln Ile Asp
TTT ACA CAA GGG GTA ATG GGG TGG CAT GTA ACT GGA AAT GCA GAC GTA CAA CAA ATA GAT

              1085             1090             1095             1100
Gly Val Ser Val Leu Val Leu Ser Asn Trp Ser Ala Gly Val Ser Gln Asn Val His Leu
GGT GTT TCT GTA TTG GTT CTA TCT AAT TGG AGT GCT GGC GTA TCT CAA AAT GTC CAT CTC

              1105             1110             1115             1120
Gln His Asn His Gly Tyr Val Leu Arg Val Ile Ala Lys Lys Glu Gly Pro Gly Asn Gly
CAA CAT AAT CAT GGG TAT GTC TTA CGT GTT ATT GCC AAA AAA GAA GGA CCT GGA AAT GGG

              1125             1130             1135             1140
Tyr Val Thr Leu Met Asp Cys Glu Glu Asn Gln Glu Lys Leu Thr Phe Thr Ser Cys Glu
TAT GTC ACG CTT ATG GAT TGT GAG GAG AAT CAA GAA AAA TTG ACG TTT ACG TCT TGT GAA

              1145             1150             1155             1160
Glu Gly Tyr Ile Thr Lys Thr Val Asp Val Phe Pro Asp Thr Asp Arg Val Arg Ile Glu
GAA GGA TAT ATT ACG AAG ACA GTA GAT GTA TTC CCA GAT ACA GAT CGT GTA CGA ATT GAG

              1165             1170             1175             1180
Ile Gly Glu Thr Glu Gly Ser Phe Tyr Ile Glu Ser Ile Glu Leu Ile Cys Met Asn Glu
ATA GGC GAA ACC GAA GGT TCG TTT TAT ATC GAA AGC ATT GAA TTA ATT TGC ATG AAC GAG
```

13

## SEQUENCE LISTING

SEQ ID No. 2
SEQUENCE TYPE: Nucleotide with corresponding protein
SEQUENCE LENGTH: 2061 base pairs
STRANDEDNESS: single
TOPOLOGY: linear
MOLECULE TYPE: genomic DNA
ORIGINAL SOURCE ORGANISM: Bacillus thuringiensis var. morrisoni (PS71M3)
PROPERTIES: Dipteran-active toxin gene

```
               5                  10                 15                 20
Met Asn Pro Tyr Gln Asn Lys Asn Glu Tyr Glu Ile Phe Asn Ala Pro Ser Asn Gly Phe
ATG AAT CCA TAT CAA AAT AAG AAT GAA TAT GAA ATA TTC AAT GCT CCA TCC AAT GGT TTT

              25                 30                 35                 40
Ser Lys Ser Asn Asn Tyr Ser Arg Tyr Pro Leu Ala Asn Lys Pro Asn Gln Pro Leu Lys
AGC AAG TCT AAT AAC TAT TCT AGA TAT CCA TTA GCA AAT AAG CCA AAT CAA CCA CTG AAA

              45                 50                 55                 60
Asn Thr Asn Tyr Lys Asp Trp Leu Asn Val Cys Gln Asp Asn Gln Gln Tyr Gly Asn Asn
AAC ACG AAT TAC AAA GAT TGG CTC AAT GTG TGT CAA GAT AAT CAA CAA TAT GGC AAT AAT

              65                 70                 75                 80
Ala Gly Asn Phe Val Ser Ser Glu Thr Ile Val Gly Val Ser Ala Gly Ile Ile Val Val
GCG GGG AAT TTT GTT AGT TCT GAA ACT ATT GTT GGA GTT AGT GCA GGT ATT ATT GTA GTA

              85                 90                 95                100
Gly Thr Met Leu Gly Ala Phe Ala Ala Pro Val Leu Ala Ala Gly Ile Ile Ser Phe Gly
GGA ACT ATG TTA GGA GCT TTT GCT GCC CCT GTC TTA GCT GCA GGT ATA ATA TCT TTT GGG

             105                110                115                120
Thr Leu Leu Pro Ile Phe Trp Gln Gly Ser Asp Pro Ala Asn Val Trp Gln Asp Leu Leu
ACT TTG TTG CCG ATC TTT TGG CAA GGA TCT GAC CCT GCA AAT GTT TGG CAG GAT TTG TTA

             125                130                135                140
Asn Ile Gly Gly Arg Pro Ile Gln Glu Ile Asp Lys Asn Ile Ile Asn Val Leu Thr Ser
AAC ATC GGA GGA AGG CCT ATA CAA GAA ATA GAT AAA AAC ATA ATT AAT GTA CTA ACT TCT

             145                150                155                160
Ile Val Thr Pro Ile Lys Asn Gln Leu Asp Lys Tyr Gln Glu Phe Phe Asp Lys Trp Glu
ATC GTA ACA CCT ATA AAA AAT CAA CTT GAT AAA TAT CAA GAA TTT TTC GAT AAA TGG GAG

             165                170                175                180
Pro Ala Arg Thr His Ala Asn Ala Lys Ala Val His Asp Leu Phe Thr Thr Leu Glu Pro
CCA GCA CGT ACA CAC GCT AAT GCT AAA GCA GTA CAT GAT CTC TTT ACT ACC TTA GAA CCT

             185                190                195                200
Ile Ile Asp Lys Asp Leu Asp Met Leu Lys Asn Asn Ala Ser Tyr Arg Ile Pro Thr Leu
ATA ATA GAT AAA GAT TTA GAT ATG TTA AAA AAT AAT GCT AGC TAT CGA ATA CCA ACA CTC

             205                210                215                220
Pro Ala Tyr Ala Gln Ile Ala Thr Trp His Leu Asn Leu Leu Lys His Ala Ala Thr Tyr
CCT GCA TAT GCA CAA ATA GCT ACT TGG CAC TTG AAT TTA TTA AAA CAT GCT GCT ACC TAT

             225                230                235                240
Tyr Asn Ile Trp Leu Gln Asn Gln Gly Ile Asn Pro Ser Thr Phe Asn Ser Ser Asn Tyr
TAC AAT ATA TGG CTG CAA AAT CAA GGT ATA AAT CCA AGT ACT TTC AAT TCA TCT AAT TAC

             245                250                255                260
Tyr Gln Gly Tyr Leu Lys Arg Lys Ile Gln Glu Tyr Thr Asp Tyr Cys Ile Gln Thr Tyr
TAT CAG GGC TAT TTA AAA CGT AAA ATA CAA GAA TAT ACT GAC TAT TGT ATA CAA ACG TAC

             265                270                275                280
Asn Ala Gly Leu Thr Met Ile Arg Thr Asn Thr Asn Ala Thr Trp Asn Met Tyr Asn Thr
AAT GCA GGA CTA ACT ATG ATT AGA ACT AAT ACT AAC GCA ACA TGG AAT ATG TAT AAT ACT
```

```
                    285                 290                 295                 300
Tyr Arg Leu Glu Met Thr Leu Thr Val Leu Asp Leu Ile Ala Ile Phe Pro Asn Tyr Asp
TAC CGT TTA GAA ATG ACT CTA ACT GTG TTA GAT CTT ATT GCT ATT TTT CCA AAT TAT GAC

                    305                 310                 315                 320
Pro Glu Lys Tyr Pro Ile Gly Val Lys Ser Glu Leu Thr Arg Glu Val Tyr Thr Asn Val
CCA GAA AAA TAT CCA ATA GGA GTT AAA TCT GAA CTT ACC AGA GAA GTT TAT ACG AAT GTT

                    325                 330                 335                 340
Asn Ser Asp Thr Phe Arg Thr Ile Thr Glu Leu Glu Asn Gly Leu Thr Arg Asn Pro Thr
AAT TCA GAT ACA TTT AGA ACC ATA ACA GAA CTA GAA AAT GGA TTA ACT AGA AAT CCT ACA

                    345                 350                 355                 360
Leu Phe Thr Trp Ile Asn Gln Gly Arg Phe Tyr Thr Arg Asn Ser Arg Asp Ile Leu Asp
TTA TTT ACT TGG ATA AAC CAA GGG CGT TTT TAC ACA AGA AAT TCT CGA GAC ATT CTT GAT

                    365                 370                 375                 380
Pro Tyr Asp Ile Phe Ser Phe Thr Gly Asn Gln Met Ala Phe Thr His Thr Asn Asp Asp
CCT TAT GAT ATT TTT TCT TTT ACA GGT AAC CAG ATG GCC TTT ACA CAT ACT AAT GAT GAT

                    385                 390                 395                 400
Arg Asn Ile Ile Trp Gly Ala Val His Gly His Ile Ile Ser Gln Asp Thr Ser Lys Val
CGC AAC ATA ATC TGG GGA GCG GTT CAT GGA CAT ATT ATT TCT CAA GAC ACA TCC AAA GTA

                    405                 410                 415                 420
Phe Pro Phe Tyr Arg Asn Lys Pro Ile Asp Lys Val Glu Ile Val Arg His Arg Glu Tyr
TTT CCT TTT TAT AGA AAC AAA CCT ATT GAT AAG GTC GAA ATT GTC AGA CAT AGA GAG TAC

                    425                 430                 435                 440
Ser Asp Ile Ile Tyr Glu Met Ile Phe Phe Ser Asn Ser Ser Glu Val Phe Arg Tyr Ser
TCA GAT ATA ATA TAT GAA ATG ATA TTT TTT TCG AAT AGC AGT GAA GTA TTT CGA TAT TCA

                    445                 450                 455                 460
Ser Asn Ser Thr Ile Glu Asn Asn Tyr Lys Arg Thr Asp Ser Tyr Met Ile Pro Lys Gln
TCC AAT TCA ACA ATA GAA AAT AAT TAT AAA AGA ACT GAT TCT TAT ATG ATT CCA AAA CAA

                    465                 470                 475                 480
Thr Trp Lys Asn Lys Glu Tyr Gly His Thr Leu Ser Tyr Ile Lys Thr Asp Asn Tyr Ile
ACA TGG AAA AAT AAA GAA TAT GGT CAT ACT CTA TCG TAT ATA AAA ACT GAT AAT TAT ATA

                    485                 490                 495                 500
Phe Ser Val Val Arg Glu Arg Arg Arg Val Ala Phe Ser Trp Thr His Thr Ser Val Asp
TTT TCA GTA GTT AGA GAA AGA AGA AGA GTT GCA TTT AGT TGG ACA CAT ACT AGT GTT GAT

                    505                 510                 515                 520
Phe Gln Asn Thr Ile Asp Leu Asp Asn Ile Thr Gln Ile His Ala Leu Lys Ala Leu Lys
TTC CAA AAT ACA ATA GAT TTA GAT AAC ATC ACC CAA ATC CAC GCT CTA AAA GCT TTG AAG

                    525                 530                 535                 540
Val Ser Ser Asp Ser Lys Ile Val Lys Gly Pro Gly His Thr Gly Gly Asp Leu Val Ile
GTA AGT TCT GAT TCG AAA ATT GTG AAA GGT CCT GGT CAC ACA GGT GGA GAC TTG GTA ATT

                    545                 550                 555                 560
Leu Lys Asp Ser Met Asp Phe Arg Val Arg Phe Leu Lys Asn Val Ser Arg Gln Tyr Gln
CTT AAA GAT AGT ATG GAT TTT AGA GTT AGA TTT TTA AAA AAT GTT TCT CGA CAA TAT CAA

                    565                 570                 575                 580
Val Arg Ile Arg Tyr Ala Thr Asn Ala Pro Lys Thr Thr Val Phe Leu Thr Gly Ile Asp
GTA CGT ATT CGT TAT GCT ACT AAT GCT CCA AAG ACA ACA GTA TTC TTA ACC GGA ATA GAT
```

16

```
                585              590              595              600
      Thr Ile Ser Val Glu Leu Pro Ser Thr Thr Ser Arg Gln Asn Pro Asn Ala Thr Asp Leu
      ACT ATA AGT GTG GAG CTC CCT AGT ACC ACT TCC CGC CAA AAC CCA AAT GCT ACA GAT TTA

                605              610              615              620
      Thr Tyr Ala Asp Phe Gly Tyr Val Thr Phe Pro Arg Thr Val Pro Asn Lys Thr Phe Glu
      ACA TAT GCA GAT TTT GGA TAT GTA ACA TTT CCA AGA ACA GTT CCA AAT AAA ACA TTT GAA

                625              630              635              640
      Gly Glu Asp Thr Leu Leu Met Thr Leu Tyr Gly Thr Pro Asn His Ser Tyr Asn Ile Tyr
      GGA GAA GAC ACT TTA TTA ATG ACC TTA TAT GGT ACA CCA AAT CAT TCA TAT AAT ATA TAT

                645              650              655              660
      Ile Asp Lys Ile Glu Phe Ile Pro Ile Thr Gln Ser Val Leu Asp Tyr Thr Glu Lys Gln
      ATT GAC AAA ATC GAA TTT ATT CCA ATC ACT CAA TCT GTA TTA GAT TAT ACA GAG AAG CAA

                665              670              675              680
      Asn Ile Glu Lys Thr Gln Lys Ile Val Asn Asp Leu Phe Val Asn *** Asn Lys Val Leu
      AAT ATA GAA AAA ACA CAG AAA ATA GTG AAT GAT TTA TTT GTT AAT TAA AAC AAA GTT CTT

                685
      Thr Lys Ile Asp Ser Met Ala
      ACT AAA ATA GAT AGT ATG GCT
```

## Claims

1. DNA encoding a <u>Bacillus thuringiensis</u> toxin having all or part of the amino-acid sequence shown in Sequence ID No. 1 or Sequence ID No. 2.

2. DNA according to claim 1, having all or part of the nucleotide sequence shown in Sequence ID No. 1 or Sequence ID No. 2.

3. A toxin, active against dipteran insects, having all or part of the amino-acid sequence shown in Sequence ID No. 1 or Sequence ID No. 2.

4. A recombinant DNA transfer vector comprising DNA according to claim 1 or claim 2.

5. A prokaryotic or eukaryotic host into which a DNA transfer vector according to claim 4 has been transferred and replicated.

6. A microorganism which is a bacterial host transformed to express a <u>Bacillus thuringiensis</u> toxin having all or part of the amino-acid sequence shown in Sequence ID No. 1 or Sequence ID No. 2.

7. A microorganism according to claim 6, which is a pigmented bacterium, yeast or fungus.

8. A microorganism according to claim 6 or claim 7, which is a species of <u>Pseudomonas</u>, <u>Azotobacter</u>, <u>Erwinia</u>, <u>Serratia</u>, <u>Klebsiella</u>, <u>Rhizobium</u>, <u>Rhodopseudomonas</u>, <u>Methylophilius</u>, <u>Agrobacterium</u>, <u>Acetobacter</u>, <u>Alcaligenes</u>, <u>Bacillus</u> or <u>Streptomyces</u>; a prokaryote selected from Enterobacteriaceae, Bacillaceae, Rhizobiaceae, Spirillaceae, Lactobacillaceae, Pseudomonadaceae, Azotobacteraceae, Nitobacteraceae and Actinomycetales; or a lower eukaryote selected from Phycomycetes, Ascomycetes and Basidiomycetes.

9. A microorganism according to claim 8, which is of the species <u>Pseudomonas</u>, e.g. <u>Pseudomonas fluorescens</u>, and the toxin has the amino-acid sequence shown in Sequence ID No. 1 or Sequence ID No. 2.

10. A microorganism according to claim 8 or claim 9, wherein said species is pigmented and phylloplane-adherent.

11. A microorganism according to claim 6, which is Escherichia coli transformed with a plasmid vector containing a Bacillus thuringiensis toxin gene encoding the toxin.

12. A microorganism according to claim 11, which has the characteristics of Escherichia coli (NM-522)(pMYC1625), NRRL B-18644, or Escherichia coli (NM522)(pMYC1636), NRRL B-18693.

13. A microorganism according to any of claims 6 to 12, in the form of substantially intact cells containing the toxin intracellularly.

14. A microorganism according to claim 13, as obtained by treatment of the cells with iodine or other chemical or physical means to prolong their insecticidal activity in the environment.

15. A method for controlling dipteran insects, which comprises administering to the insects or to their environment, e.g. the rhizosphere, the phylloplane or a body of water, a microorganism according to any of claims 6 to 14.

16. Plasmid pMYC1625 or plasmid pMYC1636, as available in the microorganisms identified in claim 12.

Figure 1a.

NH2

450

600

HOOC

550

○ KD Hydrophilicity >=1.3

◇ KD Hydrophobicity >=1.3

EP 0 457 498 A2

HOOC

660

600

450    NH2

KD Hydrophobicity >-1.9

KD Hydrophilicity >-1.9

Figure 1b.